# EUROPÄISCHE PATENTANMELDUNG

(11) **EP 3 093 336 A1**
(43) Veröffentlichungstag der Anmeldung: **16.11.2016**
(21) Anmeldenummer: 15167132.8
(22) Anmeldetag: 11.05.2015
(51) Int. Cl.: C12M 1/06, B01F 3/04, C12P 19/04, C12M 1/00, C12M 1/02

(54) **FERMENTER ZUR HERSTELLUNG EINES STRUKTURVISKOSEN MEDIUMS**

(71) Anmelder: Wintershall Holding GmbH, 34119 Kassel (DE)
(72) Erfinder: Scholz, Alexander, 67112 Mutterstadt (DE); Lehr, Florian, 67365 Schwegenheim (DE); Rollie, Sascha, 68167 Mannheim (DE); Fleck, Christian, 69207 Sandhausen (DE); Hofinger, Julia, 67063 Ludwigshafen (DE); Pollmer, Nadja, 67281 Bissersheim (DE); Dienes, Christian, 76857 Wernersberg (DE); Schreiber, Michael, 68167 Mannheim (DE)
(74) Vertreter: Maiwald Patentanwalts GmbH

(57) **Zusammenfassung**

Fermenter zur Herstellung eines strukturviskosen Mediums mit einem Tankvolumen und einer Rühranordnung mit einem verbesserten Verteilungsvermögen bzw. einer gleichmäßigeren Scherbeanspruchung.

## Beschreibung

### Gebiet der Erfindung

Die vorliegende Erfindung betrifft eine Vorrichtung zur Fermentierung einer Brühe für die Herstellung eines strukturviskosen Mediums, insbesondere einen Fermenter zur Herstellung von Polysacchariden bzw. Glucanen, der für die Durchmischung des strukturviskosen Mediums eine gleichmäßige Scherbeeinflussung bzw. einen großen Bereich mit niedriger Viskosität ermöglicht.

### Hintergrund der Erfindung

Für die Herstellung von Polysacchariden bzw. Glucanen können Fermenter verwendet werden, in denen das während der Herstellung erzeugte strukturviskose Medium in dem Fermenter auch bewegt wird. Eine derartige Bewegung kann beispielsweise durch eine Rühranordnung hervorgerufen werden. Die bei der Herstellung von Polysacchariden bzw. Glucanen üblicherweise auftretenden strukturviskosen Medien haben dabei jedoch eine viskositätsbezogene Eigenschaft, die den Rührprozess in Abhängigkeit von einer lokalen Scherbeanspruchung in einem Fermenter beeinflusst.

Als strukturviskos wird ein Fluid bzw. ein Medium bezeichnet, wenn die Eigenschaft des Fluids bei hohen Scherkräften eine abnehmende Viskosität zeigt. Das heißt, je stärker die Scherung ist, die auf das Fluid wirkt, desto weniger viskos/zähflüssig wird es. Ein derartiges Fluid wird synonym auch als scherverdünnend bezeichnet. Eine derartige Abnahme der Viskosität bei Scherbeanspruchung entsteht zum Beispiel durch eine Strukturänderung im Fluid, die dafür sorgt, dass die einzelnen Fluidpartikel, beispielsweise Polymerketten, besser aneinander vorbeigleiten können. Da in einem strukturviskosen Fluid bzw. Medium die Viskosität bei wachsender Scherung nicht konstant bleibt, wird das Fluid üblicherweise als nicht-Newton'sches Fluid klassifiziert, so dass die üblichen Strömungsansätze für Newton'sche Fluide darauf nicht angewendet werden können. Daher gelten die üblichen Strömungsbetrachtungen von Fluiden nicht mehr und eine Durchmischung kann nicht mehr mit einfachen Rührergeometrien erreicht werden.

Wird nun ein solches strukturviskoses Medium gerührt, so führt die lokal Scherbeanspruchung zu einer lokalen Verringerung der Viskosität, so dass lokal eine höhere Fließfähigkeit des strukturviskosen Mediums auftritt. Dies erfordert andere Rührergeometrien als für beispielsweise Newton'sche Fluide, um durch die Rührergeometrie eine gleichmäßige Umwälzung und Verteilung innerhalb eines Fermentervolumens zu erreichen.

Aus dem Stand der Technik sind unterschiedliche Rührergeometrien bekannt. Beispielsweise wird in "Xanthan Production in Stirred Tank Fermenters: Oxygen Transfer and Scale-up" von Holger Herbst, Adrian Schumpe und Wolf-Dieter Deckwer, ein Reaktor beschrieben, bei dem das Durchmesserverhältnis von Rührer und Rührvolumen maximal 0,7 beträgt.

In "Performance of the Scaba 6SRGT Agitator in Mixing of Simulated Xanthan Gum Broths" von Enrique Galindo und Alvin W. Nienow, wird beispielsweise eine Rührergeometrie beschrieben, die einen Durchmesser von 0,2 m aufweist und ein Durchmesserverhältnis von Rührer zu Rührvolumen von weniger als 0,5 aufweist.

In "Mass Transfer Coefficient in Stirred Tank Reactors for Xanthan Gum Solutions" von Felix Garcia-Ochoa und Emilio Gomez, wird ein Rührer beschrieben, der für ein 20-Liter-Volumen verwendet wird und einen Durchmesser von 10 cm aufweist, wobei der Durchmesser des Tanks 30 cm beträgt, wodurch sich ein Durchmesserverhältnis von Rührer zu Tankvolumen von 0,3 ergibt.

In "Oxygen Transfer and Uptake Rates during Xanthan Gum Production" von F. Garcia-Ochoa, E. Gomez Castro und V. E. Santos wird eine Rührertankgeometrie beschrieben, bei der ein Durchmesserverhältnis von Rührer zu Tankdurchmesser 0,42 beträgt.

In "Effect of Mixing Behavior on Gas-Liquid Mass Transfer in Highly Viscose, Stirred Non-Newtonian Liquids" von Hans-Jürgen Henzler und Gerd Obernosterer wird ein Durchmesserverhältnis von Rührer zu Tank von maximal 0,65 beschrieben.

In WO 2004/058377 wird eine Rührergeometrie beschrieben, bei der in einem Tank ein Leitblechzylinder vorgesehen ist, wobei sich der Rührer nur bis zu der Leitblechgeometrie in dem Tankvolumen erstreckt.

In EP 1 258 502 sind einfache Rührergeometrien beschrieben für die Herstellung eines Alkoxyl-Verbundes.

Es hat sich herausgestellt, dass all diese zuvor beschriebenen Rührergeometrien für die Verrührung und die gleichmäßige Umsetzung eines strukturviskosen Mediums in einem Fermenter nicht geeignet sind, um eine hinreichend gleichmäßige Scherbeeinflussung zu gewährleisten bzw. einen hinreichend hohen Bereich mit niedriger Viskosität bereitzustellen.

### Gegenstand der vorliegenden Erfindung

Vor dem Hintergrund des bekannten Standes der Technik kann es als eine Aufgabe betrachtet werden, eine gleichmäßige Scherbeeinflussung bzw. einen hohen Bereich mit niedriger Viskosität in einem strukturviskosen Medium innerhalb eines Fermenters mit einer Rühranordnung bereitzustellen.

Diese Aufgabe wird gelöst durch den Gegenstand der unabhängigen Ansprüche. Vorteilhafte Weiterbildungen werden in den abhängigen Ansprüchen verkörpert.

Gemäß einer Ausführungsform der Erfindung wird ein Fermenter zur Herstellung eines strukturviskosen Mediums bereitgestellt, wobei der Fermenter umfasst: ein Tankvolumen und eine Rühranordnung mit einem ersten Rührorgan mit wenigstens einem Rührflügel, einem zweiten Rührorgan mit wenigstens einem Rührflügel und einer Drehachse, wobei das erste Rührorgan und das zweite Rührorgan an der Drehachse derart festgelegt sind, dass sie sich mit der Drehachse drehen und axial beabstandet sind, wobei die Drehachse bei bestimmungsgemäßem Gebrauch im Wesentlichen parallel in Bezug auf die Richtung des Erdschwerefeldes ausgerichtet ist, und wobei das Tankvolumen im Bereich der Rührorgane im Wesentlichen die Form eines Kreiszylinders aufweist und sich die Drehachse im Wesentlichen auf der Mittelachse des Kreiszylinders befindet, wobei sich die Rührflügel des ersten Rührorgans und die Rührflügel des zweiten Rührorgans bis mindestens zu einem 0,8-fachen des Abstandes zwischen Mittelachse des Kreiszylinders und einer Wand des Kreiszylinders erstrecken, sodass sich ein Verhältnis (d/D) von Rührorgandurchmesser (d) zu Innendurchmesser (D) des Tanks von mindestens 0,8 ergibt.

Auf diese Weise kann innerhalb eines Fermenters zur Herstellung eines strukturviskosen Mediums eine gleichmäßige Scherbeeinflussung des strukturviskosen Mediums erreicht werden und insbesondere im strukturviskosen Medium ein hoher Bereich mit niedriger Viskosität erzielt werden. Durch den verhältnismäßig großen Durchmesser des Rührorgans, welches bis nah an die Innenwand des Tankvolumens heranragt, kann ein großer Bereich des strukturviskosen Mediums einer Scherbeanspruchung unterzogen werden, so dass in großen Bereichen die Viskosität im strukturviskosen Medium abnimmt bzw. verringert wird. Durch die Anordnung eines ersten Rührorgans und eines zweiten Rührorgans über- bzw. untereinander kann ferner nicht nur in radialer Richtung, sondern auch in axialer Richtung in einem großen Bereich eine Scherbeanspruchung des strukturviskosen Mediums erreicht werden, so dass sich in einem verhältnismäßig großen Bereich bei einer Betätigung bzw. Drehung der Rührorgane mit der Drehachse die Viskosität verringert. Es sei verstanden, dass obgleich die Rührorgane auch nur einen einzigen Rührflügel aufweisen können, der Durchmesser des Rührorgans als der Kreis verstanden wird, der durch die äußerste Spitze des auch nur einzigen Rührflügels gezeichnet wird. Es sei dabei verstanden, dass die Rührflügel in ihrer radialen Erstreckungsrichtung ausgehend von der Drehachse eine einheitliche Form, d.h. keine sich ändernde Querschnittsform des Rührblattes aufweisen können, jedoch auch über radial von der Drehachse wegragende Stäbe mit der Drehachse verbunden sein können.

Gemäß einer Ausführungsform wird ein Fermenter zur Produktion eines extrazellulären, viskositätserhöhenden Polysaccharides bereitgestellt, welches in Lösung scherverdünnendes Verhalten zeigt, wobei das Viskositätsverhalten der produzierten Fermentationsbrühe durch den Potenzansatz nach Ostwald de Waele in einem Scherratenbereich von 1 bis 150 s-1 beschrieben werden kann und im Laufe des Prozesses scherratenabhängige Mindest-Viskositätswerte erreicht, die durch einen Konsistenzfaktor von K = 11,98 Pas² und einen Fließindex von n = 0,1 beschrieben werden können. Der Potenzansatz nach Ostwald de Waele ist beschrieben in Zlokarnik, M. (2000) Dimensionsanalytische Behandlung veränderlicher Stoffgrößen, in Scale-up: Modellübertragung in der Verfahrenstechnik, Wiley-VCH Verlag GmbH & Co. KGaA, Weinheim.

Gemäß einer Ausführungsform der Erfindung ist das erste Rührorgan und das zweite Rührorgan derart ausgestaltet, dass sich bei einer Drehung der Drehachse bei einem zu rührenden scherverdünnenden Medium an den radial äußeren Enden der Rührflügel eine Strömung einstellt, die eine primär axiale Richtung aufweist.

Auf diese Weise kann das strukturviskose Medium in dem Fermenter nicht nur in der Ebene der Rührorgane einer Scherbeanspruchung unterzogen werden, sondern durch die primär axiale Förderrichtung auch in dem Volumen oberhalb bzw. unterhalb des Rührorgans. Auf diese Weise kann auch der Zwischenbereich zwischen den beiden Rührorganen einer Scherbeanspruchung unterzogen werden bzw. das in diesem Zwischenbereich befindliche strukturviskose Medium in den Scherbereich der beiden Rührorgane gebracht werden. Bei der Betätigung der beiden Rührorgane können sich dabei innerhalb des strukturviskosen Mediums wirbelähnliche Strömungen einstellen, wobei die Wirbel eine größere axiale Erstreckung als radiale Erstreckung aufweisen können. Mit einer axialen Erstreckung ist hier eine Erstreckung parallel zur Drehachse gemeint. Es sein verstanden, dass unter Wirbeln nicht nur geschlossene Flusslinien zu verstehen sind, sondern auch nicht geschlossene Flusslinien oder Abschnitte.

Gemäß einer Ausführungsform der Erfindung beträgt das Durchmesserverhältnis von Rührorganen zu Tankdurchmesser d/D 0,9 ± 5 %.

Auf diese Weise können die Rührorgane bis sehr nahe an die Behälterwand heranragen, um so auch in diesem Bereich eine hohe Scherbeanspruchung und eine Verminderung der Viskosität zu erreichen. Dadurch kann das strukturviskose Medium bis nah an den Wandbereich des Fermenters umgewälzt bzw. homogenisiert werden, wodurch der Fermentationsprozess begünstigt wird.

Gemäß einer Ausführungsform der Erfindung weist das erste Rührorgan neben dem ersten Rührflügel einen zweiten Rührflügel auf, wobei sich der erste Rührflügel und der zweite Rührflügel jeweils in Bezug auf die Drehachse an gegenüberliegenden Seiten der Drehachse orthogonal von der Drehachse weg erstrecken.

Auf diese Weise kann das Rührorgan im Wesentlichen symmetrisch ausgestaltet werden, mit zwei sich gegenüberliegenden Rührflügeln. Es sei verstanden, dass auch das zweite Rührorgan und jedes weitere Rührorgan eine derartige Ausgestaltung aufweisen kann. Durch die symmetrische Ausgestaltung des Rührorgans wird eine ungleichmäßige Beanspruchung der Rührorgane und der Drehachse, insbesondere deren Lagerung und deren Antrieb, vermieden.

Gemäß einer Ausführungsform der Erfindung weisen das erste Rührorgan und das zweite Rührorgan eine übereinstimmende Anzahl von wenigstens zwei Rührflügeln auf, wobei die Rührflügel des ersten Rührorgans versetzt zu den Rührflügeln des zweiten Rührorgans angeordnet sind.

Auf diese Weise kann zum einen eine gleichmäßige Beanspruchung der Rührorgane, der Drehachse, deren Lagerung und deren Antrieb erreicht werden, und zum anderen eine gleichmäßigere Scherbeanspruchung des strukturviskosen Mediums erreicht werden. Durch die versetzte Anordnung der Rührflügel durchfährt zu jedem Zeitpunkt immer nur ein Rührflügel von dem ersten und dem zweiten Rührflügel eine vertikale Ebene in dem Tankvolumen, so dass sich eine gleichmäßigere Verteilung des strukturviskosen Mediums einstellen kann.

Gemäß einer Ausführungsform der Erfindung sind die Rührflügel von dem ersten und dem zweiten Rührorgan um einen Viertelkreis zueinander versetzt angeordnet.

Auf diese Weise kann eine Vergleichmäßigung des Abstandes für eine homogenere Scherbeanspruchung durch die Rührflügel in dem strukturviskosen Medium erreicht werden.

Gemäß einer Ausführungsform der Erfindung weisen die Rührorgane jeweils drei bzw. vier gleichmäßige verteilte Rührflügel auf. Es sei verstanden, dass auch bei Rührorganen mit drei, vier oder mehr Rührflügeln diese Rührflügel zu Flügeln von benachbarten Rührorganen versetzt zueinander angeordnet sein können. Sie können insbesondere so angeordnet sein, dass sich ein Rührflügel eines Rührorgans rotationsmäßig versetzt in der Mitte zwischen zwei Rührflügeln des benachbarten Rührorgans befindet.

Gemäß einer Ausführungsform der Erfindung sind die Rührflächen des ersten Rührflügels und des zweiten Rührflügels wenigstens im Bereich der äußeren Enden der Rührflügel gegenüber der Senkrechten im Wesentlichen um die Erstreckungsrichtung des entsprechenden Rührflügels geneigt.

Auf diese Weise kann beispielsweise erreicht werden, dass die Förderung des strukturviskosen Mediums durch die geneigten Rührblätter im Bereich der äußeren Enden axial nach unten bzw. axial nach oben erfolgt, je nach dem in welche Richtung in Bezug auf die Drehrichtung die Rührflächen der Rührflügel geneigt sind. Es sei verstanden, dass nicht nur ein einziger Rührflügel je Rührorgan, sondern alle Rührflügel des jeweiligen Rührorgans einheitlich geneigte Rührflächen aufweisen können.

Gemäß einer Ausführungsform der Erfindung sind die Flächen bzw. Rührflächen des ersten Rührflügels und des zweiten Rührflügels gegenüber der Senkrechten (parallel zur Drehachse) zwischen 30° und 60°, insbesondere zwischen 40° und 50°, insbesondere um 45° ± 2° geneigt.

Auf diese Weise kann ein optimales Verhältnis einer Massenverdrängung des strukturviskosen Mediums während eines Rührprozesses bei gleichzeitiger Scherbeanspruchung erreicht werden.

Gemäß einer Ausführungsform der Erfindung variiert die Neigung der Rührflächen über die Erstreckungsrichtung von der Drehachse in Richtung der Tankinnenwände, so dass unter Berücksichtigung der unterschiedlichen Bahngeschwindigkeiten in Abhängigkeit vom Abstand zu der Drehachse eine gleichmäßige Scherbeanspruchung erreicht werden kann. So kann beispielsweise die Neigung der Rührflächen gegenüber der Senkrechten in der Nähe der Drehachse 60° betragen und in Richtung der Spitzen der Rührflügel bis auf 45° abnehmen.

Gemäß einer Ausführungsform der Erfindung umfasst der Fermenter ferner eine aktive Temperierfläche zum Heizen und/oder Kühlen, wobei der Strömungsverlauf entlang der Temperierfläche geführt wird.

Auf diese Weise kann der Fermentierungsprozess innerhalb des Fermenters gesteuert und je nach Anforderung an den Fermentierungsprozess beschleunigt oder abgebremst werden, nämlich durch entsprechendes Heizen bzw. Kühlen der aktiven Temperierfläche des Fermenters. Die Temperierflächen können dabei an der Tankwand vorgesehen sein, jedoch auch innerhalb des Tankvolumens angeordnet sein.

Gemäß einer Ausführungsform der Erfindung ist die Temperierfläche durch umlaufende Rohrabschnitte ausgestaltet, die in Bezug auf die Drehachse in axialer Richtung in Gruppen angeordnet sind, wobei sich eine Gruppe zwischen zwei unmittelbar übereinanderliegenden Rührorganen erstreckt.

Auf diese Weise kann eine effiziente Temperierung erreicht werden, insbesondere da durch eine axiale Bewegung des strukturviskosen Mediums während eines Rührprozesses das strukturviskose Medium entlang der Temperierflächen bzw. der Gruppen von Rohrabschnitten bewegt werden kann.

Gemäß einer Ausführungsform der Erfindung weist das Tankvolumen im Bereich der Rührorgane im Wesentlichen die Form eines Kreiszylinders auf, wobei im Kreiszylinder nach innen ragende Stromstörer vorgesehen sein können, wobei sich die Stromstörer weiter nach innen erstrecken, als sich die Rührflügel nach außen in Richtung der Wand des Tankvolumens erstrecken.

Auf diese Weise erfolgt eine radiale Überdeckung der Stromstörer mit den Rührflügeln, so dass verhindert werden kann, dass sich das gesamte Volumen des strukturviskosen Mediums in eine einheitliche Drehbewegung mit den Rührorganen versetzt, wodurch die Scherbeanspruchung abnehmen würde. Die nach innen ragenden Stromstörer bremsen eine derartige Drehbewegung des strukturviskosen Mediums ab, so dass die Scherbeanspruchung wieder erhöht wird und auf diese Weise auch die Viskosität abnimmt, wodurch sich wiederum die Durchmischung des strukturviskosen Mediums erhöht. Als Stromstörer werden dabei Strukturen verstanden, die eine erzeugte Strömung unterbrechen, umleiten oder ganz allgemein stören. In dem zuvor beschriebenen Fall wird eine Kreisströmung korrespondierend zu der Drehbewegung der Rührorgane unterbrochen bzw. gestört, so dass sich die Scherbeanspruchung im strukturviskosen Medium erhöht.

Gemäß einer Ausführungsform der Erfindung halten die Stromstörer die Rohrabschnitte beabstandet von einer Wand des Tankvolumens, wobei die Rohrabschnitte weiter nach innen im Tankvolumen angeordnet sind, als sich die Rührflügel nach außen in Richtung der Wand des Tankvolumens erstrecken.

Auf diese Weise kann gewährleistet werden, dass sich das axial bewegende strukturviskose Medium insbesondere am Ende der Rührflügel entlang der Rohrabschnitte der Temperierfläche bewegt und auf diese Weise eine Temperierung erfährt.

Gemäß einer Ausführungsform der Erfindung weist die Rühranordnung ferner ein drittes Rührorgan, ein viertes Rührorgan und ein fünftes Rührorgan auf, die zueinander beabstandet auf der Drehachse angeordnet sind, wobei jedes der Rührorgane zwei Rührflügel aufweist, die um einen Viertelkreis versetzt sind gegenüber den Rührflügeln eines benachbarten Rührorgans auf der Drehachse.

Auf diese Weise kann beispielsweise eine Rühranordnung mit fünf oder mehr Rührorganen bereitgestellt werden, die beispielsweise mit gleichen Abständen auf der Drehachse befestigt sind und sich mit dieser drehen. Die einzelnen Rührorgane können dabei auch drei, vier oder mehr Rührflügel aufweisen, wodurch der Versatz dem halben Winkel zwischen zwei benachbarten Rührflügeln eines Rührorgans entspricht. Insbesondere bei drei oder mehr Rührflügeln je Rührorgan können die Rührflügel benachbarter Rührorgane auch übereinander, das heißt nicht versetzt zueinander angeordnet sein. Durch eine derartige mehrstufige Rührerkonfiguration kann eine gleichmäßige Durchmischung und Scherbeanspruchung eines strukturviskosen Mediums auch bei größeren Tankvolumina von 10 m³ bis 1.000 m³ oder mehr erreicht werden.

Gemäß einer Ausführungsform der Erfindung sind zwischen den fünf Rührorganen vier Gruppen von Rohrabschnitten vorgesehen, wobei jeweils eine Gruppe von Rohrabschnitten zwischen zwei unmittelbar übereinanderliegenden Rührorganen angeordnet ist.

Auf diese Weise kann eine gleichmäßige Temperierung im Tankvolumen des Fermenters erreicht werden.

Gemäß einer Ausführungsform der Erfindung umfasst der Fermenter eine Gaszuführungseinrichtung, deren Mündung unterhalb der wenigstens zwei Rührorgane angeordnet ist.

Auf diese Weise kann beispielsweise Sauerstoff zur Begünstigung der Fermentierung eingebracht werden, oder ein anderes Gas eingebracht werden, um beispielsweise einen Sauerstoff im strukturviskosen Medium zu verdrängen. Die Mündungen der Gaszuführungseinrichtung können insbesondere unterhalb des Überdeckungskreises der Rührflügel angeordnet sein. Es sei verstanden, dass auch oberhalb der beiden Rührorgane eine weitere Gaszuführungseinrichtung vorhanden sein kann, insbesondere kann auch eine Gaszuführungseinrichtung zwischen zwei beliebigen Rührorganen vorgesehen sein.

Gemäß einer Ausführungsform der Erfindung sind in einer Querschnittebene eines Stromstörers wenigstens drei Rohrabschnitte in axialer Richtung angeordnet.

Auf diese Weise ergibt sich eine axial erstreckte Temperierfläche. Insbesondere können in einer Querschnittebene eines Stromstörers zwei Rohrabschnitte in radialer Richtung und vier bis fünf Rohrabschnitte in axialer Richtung angeordnet sein. Es sei jedoch verstanden, dass eine beliebige Anzahl von radial nebeneinander angeordneten Rohrabschnitten und eine beliebige Anzahl von axial nebeneinander angeordneten Rohrabschnitten vorgesehen sein kann, solange diese Gruppe von Rohrabschnitten die Bewegung der Rührorgane nicht einschränkt.

Gemäß einer Ausführungsform der Erfindung wird ein Verfahren zur Produktion eines Polysaccharides unter Verwendung eines oben beschriebenen Fermenters bereitgestellt. Die oben beschriebenen auf eine Vorrichtung bezogenen Merkmale sind analog auch auf ein entsprechendes Verfahren anwendbar.

Gemäß einer Ausführungsform der Erfindung zeigt das Polysaccharid in Lösung scherverdünnendes Verhalten, wobei das Viskositätsverhalten einer produzierten Fermentationsbrühe durch den Potenzansatz nach Ostwald de Waele in einem Scherratenbereich von 1 bis 150 s-1 beschrieben wird, wobei die durch das Verfahren produzierten Fermentationsbrühe im Laufe des Prozesses scherratenabhängige Mindest-Viskositätswerte erreicht, die durch einen Konsistenzfaktor von K = 11,98 Pas² und einen Fließindex von n = 0,1 gekennzeichnet sind.

Gemäß einer Ausführungsform der Erfindung ist das Polysaccharid ein extrazelluläres, viskositätserhöhendes Polysaccharid.

Gemäß einer Ausführungsform der Erfindung ist das Polysaccharid ein Glucan, welches insbesondere wenigstens eines von einem α-Glucan, einem β-Glucan und einem Xanthan umfasst, bzw. im Wesentlichen ein α-Glucan, ein β-Glucan oder ein Xanthan ist.

Die einzelnen oben beschriebenen Merkmale können selbstverständlich auch untereinander kombiniert werden, wodurch sich zum Teil auch vorteilhafte Wechselwirkungen einstellen können, die über die Summe der Einzelwirkungen hinausgehen.

Diese und andere Aspekte der vorliegenden Erfindung werden durch die Bezugnahme auf die hiernach beschriebenen beispielhaften Ausführungsformen erläutert und verdeutlicht.

### Kurze Beschreibung der Zeichnungen

Beispielhafte Ausführungsformen werden im Folgenden mit Bezugnahme auf die folgenden Zeichnungen beschrieben.
Figur 1 zeigt eine Schnittansicht durch einen Fermenter gemäß einer beispielhaften Ausführungsform der Erfindung.
Figur 2 zeigt ein Detail aus einer Rühranordnung gemäß einer beispielhaften Ausführungsform der Erfindung.

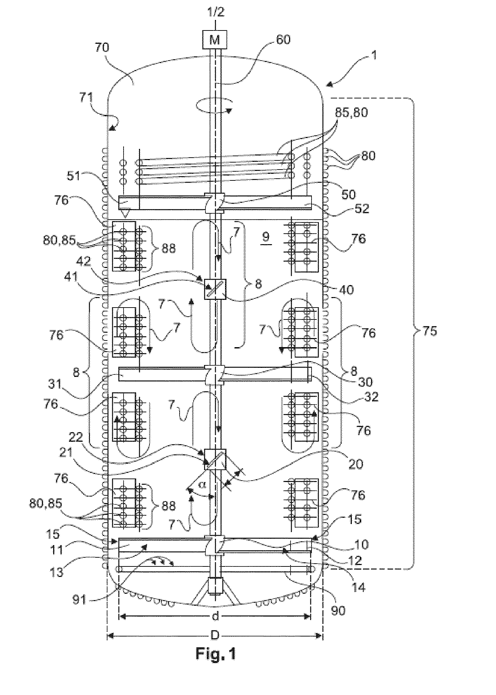

Figur 1 zeigt einen Fermenter gemäß einer beispielhaften Ausführungsform der Erfindung zur Herstellung eines strukturviskosen Mediums. Der Fermenter 1 weist dabei ein Tankvolumen 70 auf, welches durch eine Wand des Tankvolumens 71 definiert ist. In dem Tankvolumen 70 befindet sich eine Rühranordnung mit mehreren Rührorganen 10, 20, 30, 40, 50, die jeweils an einer Drehachse 60 befestigt sind und sich zusammen mit der Drehachse 60, angetrieben über einen Motor M, um die Drehachse 60 drehen können. Die Rührorgane in der in Figur 1 gezeigten Ausführungsform weisen jeweils zwei Rührflügel auf, einen ersten Rührflügel 11 und einen zweiten Rührflügel 12 für das erste Rührorgan 10, sowie analog für das zweite, dritte, vierte und fünfte Rührorgan 20, 30, 40, 50 einen jeweils ersten Rührflügel 21, 31, 41, 51 sowie einen zweiten Rührflügel 22, 32, 42, 52. Die beiden Rührflügel eines Rührorgans erstrecken sich von der Mittelachse bzw. der Drehachse 60 aus in Richtung der Wand 71 des Tankvolumens 70. In der in Figur 1 gezeigten Ausführungsform weist jedes Rührorgan zwei Rührflügel auf, die über die Erstreckungsrichtung im Wesentlichen eine konstante Neigung aufweisen. Jeder Rührflügel 11, 12 weist eine entsprechend geneigte Fläche 13, 14 auf, mit der das strukturviskose Medium bei einer Drehung der Drehachse 60 im Wesentlichen in einer axialen Richtung, das heißt mit einer Komponente parallel zu der Drehachse gefördert wird. Dabei kann bei der Förderung des strukturviskosen Mediums dieses durch die geneigten Flächen 13, 14 entweder nach oben oder nach unten gedrückt werden, je nachdem in welche Richtung sich die Drehachse 60 mit den daran befestigten Rührorganen 10 bis 50 dreht. Dabei stellt sich beispielsweise eine Strömungsrichtung 7 ein, die wirbelähnlich sein kann, wobei diese Strömung eine axiale Komponente hat, die stärker ausgebildet ist als eine radiale Komponente. Der Wirbel bzw. die wirbelähnliche Strömung ist vereinfacht durch Pfeile mit der Strömungsrichtung 7 dargestellt. In der Realität wird der Strömungsverlauf jedoch wesentlich komplexer sein, insbesondere da durch die unterschiedliche Bahngeschwindigkeit in Abhängigkeit vom Abstand zur Drehachse 60 eine unterschiedliche Kraftausübung auf das zu rührende Medium 9, das strukturviskose Medium, erfolgt. Bei der in Figur 1 gezeigten Anordnung bilden sich die Wirbel 8 im Wesentlichen so aus, dass eine axiale Umwälzung des zu rührenden Mediums 9 erfolgt, so dass auch die Bereiche zwischen den Rührorganen 10 bis 50 einer Bewegung unterzogen werden und so umgewälzt werden, dass diese auch in den Scherbereich der Rührflügel der Rührorgane gelangen. In der in Figur 1 gezeigten Ausführungsform weist jedes Rührorgan zwei Rührblätter auf, die jeweils versetzt angeordnet sind zu den Rührblättern eines unmittelbar benachbart angeordneten Rührorgans. So erstrecken sich die Rührflügel des untersten Rührorgans 10, des mittleren Rührorgans 30 und des oberen Rührorgans 50 seitlich in der Bildebene, während die Rührflügel der dazwischenliegenden Rührorgane 20 und 40 sich nach vorne aus der Bildebene heraus bzw. nach hinten in die Bildebene hinein erstrecken.

Die in Figur 1 gezeigten Rührflügel der Rührorgane weisen eine über die Erstreckungsrichtung im Wesentlichen konstante Neigung, hier mit dem Winkel α auf, der die Neigung gegenüber der Senkrechten, das heißt der Erstreckungsrichtung der Drehachse 60 angibt. Es sei verstanden, dass sich die Neigung der Rührflügel über die Erstreckungslänge der Rührflügel von der Drehachse 60 bis zur Flügelspitze verändern kann, so dass der unterschiedlichen Bahngeschwindigkeit der Rührorgane in Abhängigkeit vom Abstand zur Drehachse 60 Rechnung getragen werden kann. Insbesondere kann die Neigung der Flächen in Bezug auf die Richtung der Drehachse 60 im achsnahen Bereich größer sein als im achsfernen Bereich. Dabei sein verstanden, dass bei einer größeren Neigung die axiale Vortriebskomponente geringer ist als bei einer kleineren Neigung.

Der Füllpegel im Tankvolumen 70 befindet sich in der in Figur 1 gezeigten Ausführungsform kurz unterhalb des obersten Rührorgans, so dass das Rührorgan 50 in der Figur 1 oberhalb des zu rührenden Mediums 9 angeordnet ist. Unterhalb des untersten Rührorgans 10 ist eine Gaszuführungseinrichtung vorgesehen, deren Mündung unterhalb des untersten Rührorgans 10 liegt. Die Mündungen 91 können dabei unterhalb des Überdeckungskreises der beiden Rührflügel 11, 12 des ersten Rührorgans 10 liegen. Wenn Gas durch die Gaszuführungseinrichtung 90 in das zu rührende Medium 9 eingebracht wird, erhöht sich das Volumen des zu rührenden Mediums um die eingebrachten Gasblasen. Infolgedessen steigt der Füllstand im Tankvolumen an, so dass der Füllstand in diesem Fall bis über das oberste Rührorgan 50 ansteigen kann, so dass das oberste Rührorgan 50 zu dem Rührvorgang beiträgt. Je nachdem, in welche Richtung die Drehachse 60 mit den daran befestigten Rührorganen 10 bis 50 dreht, wird der Aufstieg der Gasblasen in dem zu rührenden Medium 9 begünstigt, nämlich wenn die Rührblätter das zu rührende Medium 9 bedingt durch die schräg stehenden Flächen der Rührflügel nach oben drücken, oder abgebremst, wenn die Rührflügel das Medium nach unten bewegen, wenn die Drehachse 60 in die entgegengesetzte Richtung dreht und die Rührflächen die Gasblasen in dem zu rührenden Medium 9 nach unten drücken.

Die Rührflügel 11, 21, 31, 41, 51; 12, 22, 32, 42, 52 erstrecken sich von der Drehachse 60 bis kurz vor die Wand 71 des Tankvolumens 70. Der Durchmesser der Rührorgane, der im Sinne der Erfindung als der Durchmesser des Überstreichungskreises des jeweiligen Rührorgans zu verstehen ist, ist annähernd so groß wie der Durchmesser des Tankvolumens 70 im Bereich eines Kreiszylinder-querschnittförmigen Abschnittes des Tankvolumens 75.

Das Durchmesserverhältnis zwischen dem Durchmesser der Rührorgane d zu dem Durchmesser des Tankvolumens D beträgt beispielsweise 0,9. Es sei verstanden, dass das Durchmesserverhältnis d/D möglichst groß gewählt werden kann, so dass eine bis in den Randbereich des Tankvolumens erfolgende Rührbewegung der Rührorgane 10 bis 50 an diesen Stellen eine Scherbeanspruchung des strukturviskosen Mediums bewirkt, so dass dort eine gute Durchmischung des zu rührenden Mediums 9 erfolgt. Das Durchmesserverhältnis d/D kann beispielsweise bis zu 0,99 betragen, sofern sichergestellt ist, dass die radial äußeren Enden 15 der Rührflügel nicht mit der Wand 71 des Tankvolumens kollidieren.

Zur Unterstützung des Fermentationsprozesses im Fermenter 1 können Temperierflächen 80 zur Verfügung gestellt werden, die das Tankvolumen 70 bzw. das darin befindliche zu rührende Medium 9 temperieren können. Diese Temperierflächen können beispielsweise in Form von äußeren Kühlschlangen an der Außenseite des Tankvolumens 70 angeordnet sein. Alternativ bzw. zusätzlich können auch Temperierflächen innerhalb des Tankvolumens 70 angeordnet sein, die sich dann beispielsweise zwischen den Rührorganen befinden. Die im Tankvolumen 70 vorgesehenen Temperierflächen können beispielsweise umlaufende Rohrabschnitte 85 sein, die beispielsweise in Form von Rohrspiralen im Tankvolumen 70 angeordnet sein können. Die umlaufenden Rohrabschnitte können dabei sowohl spiralförmig als auch kreisförmig vorgesehen sein, wobei für eine sequentielle Durchströmung die spiralförmige Anordnung vorgesehen sein kann. Es können jedoch auch kreisförmige Rohrabschnitte vorgesehen sein, die entweder parallel durchflossen werden, oder die durch eine entsprechende Kröpfung und eine Verbindung zwischen einem Rohrabschnitt und einem darüber liegenden Rohrabschnitt durch die Kröpfung sequentiell durchflossen sein können. In der in Figur 1 gezeigten Ausführungsform sind zwischen den Rührorganen Gruppen 88 von umlaufenden Rohrabschnitten vorgesehen, die in der Regel aus zwei in radialer Richtung nebeneinander liegenden Rohrabschnitten bestehen, sowie fünf unter- bzw. übereinander angeordneten Rohrabschnitten. Eine derartige Gruppe 88 von Rohrabschnitten kann durch eine entsprechende spiralförmige Führung sequentiell von einem Temperiermittel, entweder ein Kühlmittel oder einem Heizmittel durchflossen werden. Durch die Ausgestaltung der Rührflügel und die sich daraus ergebende, vorzugsweise axiale Strömung des zu rührenden Mediums 9 innerhalb des Tankvolumens wird eine Überströmung der Temperierflächen 80 bzw. der Gruppen 88 von umlaufenden Rohrabschnitten 85 erreicht, so dass in diesem Bereich eine Temperierung des zu rührenden Mediums 9 erfolgen kann. Durch die Temperierung kann der Fermentationsprozess gesteuert werden

Um zu verhindern, dass die Rotation der Rührorgane 10 bis 50 das zu rührende Medium in eine gesamte rotierende Bewegung versetzt, so dass sich das zu rührende Medium im Wesentlichen nicht mehr in Bezug auf die Rührorgane bewegt, können im Tankvolumen 70 Stromstörer 76 vorgesehen sein. Diese Stromstörer können beispielsweise Paddel oder Bleche sein, die sich von der Wand 71 des Tankvolumens 70 nach innen erstrecken, beispielsweise in Richtung der Drehachse. Es sei verstanden, dass sich die Stromstörer 76 auch vertikal und/oder horizontal geneigt in das Tankvolumen 70 hinein erstrecken können und nicht zwingend auf die Drehachse 60 zeigen müssen. Die Stromstörer können unmittelbar an der Wand 71 des Tankvolumens 70 befestigt sein oder aber durch Abstandhalter in das Tankvolumen 70 hineinragen. Die Stromstörer überdecken sich radial dabei mit den Rührflügeln der Rührorgane, so dass eine radiale Überdeckung von Stromstörern 76 und Rührflügeln 11, 21, 31, 41, 51 etc. vorliegt. Auf diese Weise wird eine Rotationsbewegung des zu rührenden Mediums 9 unterbrochen, bzw. gestört und so die Relativbewegung der Rührflügel in Bezug auf das zu rührende Medium 9 gewährleistet. Folglich kann durch die Rührorgane eine Scherbeanspruchung auf das zu rührende Medium 9 aufrechterhalten werden, wodurch sich das zu rührende Medium an dieser Stelle verdünnt und besser fließfähig wird.

Die Stromstörer 76 können dabei auch als Haltestrukturen für die Temperierflächen dienen. Insbesondere können die Stromstörer als Haltestrukturen für die Gruppen von umlaufenden Rohrabschnitten dienen und diese positionieren. Dabei können sich sowohl die Stromstörer 76 als auch die Gruppen 88 von Rohrabschnitten 85 beliebig weit in den Zwischenraum zwischen den Rührorganen hinein erstrecken, solange sie die Rotation der Rührorgane um die Drehachse 60 nicht einschränken oder behindern.

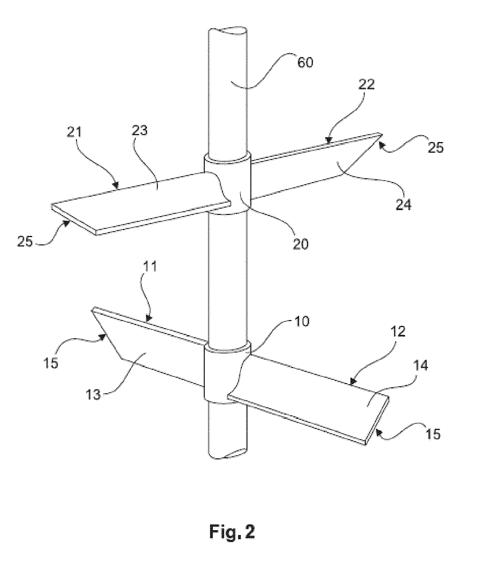

Figur 2 zeigt einen Ausschnitt aus einer Rühranordnung, die aus der Drehachse 60 sowie einem ersten Rührorgan 10 und einem zweiten Rührorgan 20 aufgebaut ist. Es sei verstanden, dass weitere Rührorgane über und unter dem ersten bzw. zweiten Rührorgan hier nicht ausgeschlossen sind. Jedes der beiden Rührorgane 10, 20 weist dabei einen ersten Rührflügel 11 bzw. 21 sowie einen zweiten Rührflügel 12 bzw. 22 auf. Die Rührflügel sind in der in Figur 2 gezeigten Anordnung um etwa 45° gegenüber der Erstreckungsrichtung der Drehachse 60 geneigt. Dadurch stehen die Flächen 13 bzw. 14 und 23 bzw. 24 schräg und können je nach Drehrichtung das zu rührende Medium 9 entweder nach oben oder nach unten beschleunigen.

Durch die aufgebrachten Scherkräfte wird das strukturviskose Medium dünnflüssiger und somit fließfähiger, so dass eine Durchmischung verbessert wird. Die äußeren Enden 15 bzw. 25 erstrecken sich dabei bis kurz vor die Wand 71 des Tankvolumens 70, welches in Figur 2 jedoch nicht gezeigt ist.

Obwohl in Figur 2 die beiden Rührorgane 10, 20 jeweils zwei sich auf gegenüberliegenden Seiten weg erstreckende Rührflügel aufweisen, können die Rührorgane 10, 20 auch drei, vier oder mehr Rührflügel aufweisen. Diese Rührflügel können dabei gleichmäßig entlang des Umfangs verteilt sein, so dass ein im Wesentlichen symmetrisches Rührorgan zur Verfügung steht.

In Figur 2 sind die Rührflügel des ersten Rührorgans 11, 12 gegenüber den Rührflügeln des zweiten Rührorgans 21, 22 versetzt angeordnet. In Figur 2 ist insbesondere ein Versatz um einen Viertelkreis dargestellt. Es sei jedoch verstanden, dass der Versatz auch unterschiedlich groß sein kann, so dass beispielsweise bei drei vorhandenen Rührorganen auf der Drehachse der Versatz von benachbarten Rührorganen jeweils 60° sein kann, so dass ein fortgesetzter Versatz von Rührorgan zu Rührorgan jeweils weitere 60° beträgt.

Insbesondere für den Fall, dass bei einem Rührorgan oder mehreren Rührorganen mehr als zwei Rührflügel vorgesehen sind, können die Rührflügel auch übereinander angeordnet sein, das heißt ohne Versatz bei benachbarten Rührorganen.

Es sollte angemerkt werden, dass die vorliegende Erfindung insbesondere auch bei strukturviskosen Medien Verwendung finden kann, die für die Förderung von Erdöl dienen können, beispielsweise Xanthan, Glucanen, insbesondere α- und β-Glucanen.

### Bezugszeichenliste

- 1: Fermenter; Rührer für strukturviskose Medien bei einer Fermentation
- 7: Strömungsrichtung
- 8: Wirbel
- 9: zu rührendes Medium
- 10: erstes Rührorgan
- 11: erster Rührflügel des ersten Rührorgans
- 12: zweiter Rührflügel des ersten Rührorgans
- 13: geneigte Fläche des ersten Rührflügels des ersten Rührorgans
- 14: geneigte Fläche des zweiten Rührflügels des ersten Rührorgans
- 15: radial äußeres Ende der Rührflügel des ersten Rührorgans
- 20: zweites Rührorgan
- 21: erster Rührflügel des zweiten Rührorgans
- 22: zweiter Rührflügel des zweiten Rührorgans
- 23: geneigte Fläche des ersten Rührflügels des zweiten Rührorgans
- 24: geneigte Fläche des zweiten Rührflügels des zweiten Rührorgans
- 25: radial äußeres Ende der Rührflügel des zweiten Rührorgans
- 30: drittes Rührorgan
- 31: erster Rührflügel des dritten Rührorgans
- 32: zweiter Rührflügel des dritten Rührorgans
- 40: viertes Rührorgan
- 41: erster Rührflügel des vierten Rührorgans
- 42: zweiter Rührflügel des vierten Rührorgans
- 50: fünftes Rührorgan
- 51: erster Rührflügel des fünften Rührorgans
- 52: zweiter Rührflügel des fünften Rührorgans
- 60: Drehachse der Rühranordnung
- 70: Tankvolumen
- 71: Wand des Tankvolumens
- 75: Abschnitts des Tanks, der die Form eines Kreiszylinders aufweist
- 76: Stromstörer; Haltestruktur für umlaufende Rohrabschnitte
- 80: Temperierfläche zum Heizen und/oder Kühlen
- 85: umlaufende Rohrabschnitte
- 88: Gruppe von umlaufenden Rohrabschnitten
- 90: Gas/Sauerstoffzuführung
- 91: Mündung der Gas/Sauerstoffzuführung
- α: (alpha) Neigungswinkel der Rührflügel gegenüber der Senkrechten
- d: Außendurchmesser der Rührorgane
- D: Innendurchmesser des Tankvolumens im Bereich 75

## Patentansprüche

1. Fermenter zur Herstellung eines strukturviskosen Mediums, umfassend:
ein Tankvolumen (70) und
eine Rühranordnung mit einem ersten Rührorgan (10) mit wenigstens einem Rührflügel (11), einem zweiten Rührorgan (20) mit wenigstens einem Rührflügel (21), und einer Drehachse (60),
wobei das erste Rührorgan (10) und das zweite Rührorgan (20) an der Drehachse (60) derart festgelegt sind, dass sie sich mit der Drehachse drehen und axial beabstandet sind,
wobei die Drehachse (60) bei bestimmungsgemäßem Gebrauch im Wesentlichen parallel in Bezug auf die Richtung des Erdschwerefeldes ausgerichtet ist, und
wobei das Tankvolumen (70) im Bereich der Rührorgane (10, 20) im Wesentlichen die Form eines Kreiszylinders (75) aufweist und sich die Drehachse (60) im Wesentlichen auf der Mittelachse des Kreiszylinders (75) befindet, wobei sich die Rührflügel (11, 12) des ersten Rührorgans (10) und die Rührflügel (21, 12) des zweiten Rührorgans (20) bis mindestens zu einem 0,8-fachen des Abstandes zwischen Mittelachse des Kreiszylinders (75) und einer Wand (71) des Kreiszylinders erstrecken, sodass sich ein Verhältnis (d/D) von Rührorgandurchmesser (d) zu Innendurchmesser (D) des Tanks von mindestens 0,8 ergibt.

2. Fermenter gemäß Anspruch 1, wobei das erste Rührorgan (10) neben dem ersten Rührflügel (11) einen einen zweiten Rührflügel (12) aufweist, wobei sich der erste Rührflügel und der zweite Rührflügel in Bezug auf die Drehachse (60) an gegenüberliegenden Seiten der Drehachse orthogonal von der Drehachse weg erstrecken.

3. Fermenter gemäß einem der Ansprüche 1 und 2, wobei das erste Rührorgan (10) und das zweite Rührorgan (20) eine übereinstimmende Anzahl von wenigstens zwei Rührflügeln (11, 12; 21, 22) aufweisen, wobei die Rührflügel (11, 12) des ersten Rührorgans versetzt zu den Rührflügeln (21, 22) des zweiten Rührorgans angeordnet sind.

4. Fermenter gemäß einem der Ansprüche 1 bis 3, wobei die Rührflügel (11, 12; 21, 22) von dem ersten und dem zweiten Rührorgan (10, 20) um einen Viertelkreis zueinander versetzt angeordnet sind.

5. Fermenter gemäß einem der Ansprüche 1 bis 4, wobei Rührflächen (13, 14) des ersten Rührflügels (11) und des zweiten Rührflügels (12) wenigstens im Bereich der äußeren Enden der Rührflügel gegenüber der Senkrechten im Wesentlichen um die Erstreckungsrichtung des entsprechenden Rührflügels geneigt sind.

6. Fermenter gemäß Anspruch 5, wobei die Rührflächen (13, 14) des ersten Rührflügels (11) und des zweiten Rührflügels (12) gegenüber der Senkrechten zwischen 30° und 60°, insbesondere zwischen 40° und 50°, insbesondere 45° +/- 2° geneigt sind.

7. Fermenter gemäß einem der Ansprüche 1 bis 6, ferner umfassend eine aktive Temperierfläche (80) zum Heizen und/oder Kühlen, wobei der Strömungsverlauf (7) entlang der Temperierfläche geführt wird.

8. Fermenter gemäß Anspruch 7, wobei die Temperierfläche (80) als umlaufende Rohrabschnitte (85) ausgestaltet ist, die in Bezug auf die Drehachse (60) in axialer Richtung in Gruppen (88) angeordnet sind, wobei sich eine Gruppe zwischen zwei unmittelbar übereinanderliegenden Rührorganen (10, 20; 20, 30) erstreckt.

9. Fermenter gemäß einem der Ansprüche 1 bis 8, wobei das Tankvolumen (70) im Bereich der Rührorgane (10, 20, 30, 40, 50) im Wesentlichen die Form eines Kreiszylinders (75) aufweist, wobei im Kreiszylinder nach innen ragende Stromstörer (76) vorgesehen sind, wobei sich die Stromstörer weiter nach innen erstrecken, als sich die Rührflügel (11, 12, 21, 22) nach außen in Richtung der Wand (71) des Tankvolumens (70) erstrecken.

10. Fermenter gemäß Anspruch 9, wobei die Stromstörer die Rohrabschnitte (85) beabstandet von einer Wand (71) des Tankvolumens halten, wobei die Rohrabschnitte weiter nach innen im Tankvolumen (70) angeordnet sind, als sich die Rührflügel (11, 12, 21, 22) nach außen in Richtung der Wand (71) des Tankvolumens (70) erstrecken.

11. Fermenter gemäß Anspruch 9, wobei in einer Querschnittsebene eines Stromstörers (76) wenigstens ein Rohrabschnitt (85) in radialer Richtung und wenigstens drei Rohrabschnitte (85) in axialer Richtung angeordnet sind.

12. Fermenter gemäß einem der Ansprüche 1 bis 11, wobei die Rühranordnung ferner ein drittes Rührorgan (30), ein viertes Rührorgan (40) und ein fünftes Rührorgan (50) aufweist, die zueinander beabstandet auf der Drehachse (60) angeordnet sind, wobei jedes der Rührorgane (30, 50) zwei Rührflügel (31, 32; 51, 52) aufweist, die um einen Viertelkreis versetzt sind gegenüber den Rührflügeln (21, 22; 41, 42) eines benachbarten Rührorgans (20, 40) auf der Drehachse (60).

13. Fermenter gemäß Anspruch 12, wobei zwischen den fünf Rührorganen vier Gruppen (88) von Rohrabschnitten (85) vorgesehen sind, wobei jeweils eine Gruppe (88) von Rohrabschnitten (85) zwischen zwei unmittelbar übereinanderliegenden Rührorganen (10, 20; 20, 30; 30, 40; 40, 50) angeordnet ist.

14. Fermenter gemäß einem der Ansprüche 1 bis 13, ferner umfassend eine Gaszuführungseinrichtung (90), deren Mündung (91) unterhalb der wenigstens zwei Rührorgane (10, 20, 30, 40, 50) angeordnet ist.

15. Verfahren zur Produktion eines Polysaccharides unter Verwendung eines Fermenters nach einem der Ansprüche 1 bis 14,

16. Verfahren gemäß Anspruch 15, wobei das Polysaccharid in Lösung scherverdünnendes Verhalten zeigt, wobei das Viskositätsverhalten einer produzierten Fermentationsbrühe durch den Potenzansatz nach Ostwald de Waele in einem Scherratenbereich von 1 bis 150 s-1 beschrieben wird, wobei die durch das Verfahren produzierten Fermentationsbrühe im Laufe des Prozesses scherratenabhängige Mindest-Viskositätswerte erreicht, die durch einen Konsistenzfaktor von K = 11,98 Pas² und einen Fließindex von n = 0,1 gekennzeichnet sind.

17. Verfahren gemäß einem der Ansprüche 15 und 16, wobei das Polysaccharid ein extrazelluläres, viskositätserhöhendes Polysaccharid ist.

18. Verfahren gemäß einem der Ansprüche 15 bis 17, wobei das Polysaccharid ein Glucan ist, insbesondere wenigstens eines von einem α-Glucan, einem β-Glucan und einem Xanthan umfasst.
